(19) Europäisches Patentamt
European Patent Office
Office européen des brevets

(11) **EP 4 785 934 A1**

(12) **EUROPEAN PATENT APPLICATION**
published in accordance with Art. 153(4) EPC

(43) Date of publication:
05.08.2026 Bulletin 2026/32

(51) International Patent Classification (IPC):
*A61K 9/48* (2006.01)    *A23L 5/00* (2016.01)
*A23L 33/10* (2016.01)    *A61K 47/44* (2017.01)

(21) Application number: 24872252.2

(22) Date of filing: 25.09.2024

(52) Cooperative Patent Classification (CPC):
A23L 5/00; A23L 33/10; A61K 9/48; A61K 47/44

(86) International application number:
PCT/JP2024/034124

(87) International publication number:
WO 2025/070466 (03.04.2025 Gazette 2025/14)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 29.09.2023 JP 2023170003

(71) Applicant: Morishita Jintan Co., Ltd.
Osaka-shi, Osaka 540-8566 (JP)

(72) Inventors:
• RI, Shin
Hirakata-shi, Osaka 573-0128 (JP)
• NAGAE, Kentaro
Hirakata-shi, Osaka 573-0128 (JP)
• NISHIKAWA, Takehiro
Hirakata-shi, Osaka 573-0128 (JP)

(74) Representative: dompatent
Partnerschaft von
Patentanwälten und Rechtsanwälten mbB
Deichmannhaus am Dom
Bahnhofsvorplatz 1
50667 Köln (DE)

(54) **SEAMLESS CAPSULE FOR ORAL CAVITY**

(57)    Provided is a seamless capsule for an oral cavity in which a semi-solid substance is used as a content of the seamless capsule instead of a liquid substance, and which allows little residue to remain undissolved in the oral cavity.

A seamless capsule for an oral cavity, including: a content; and a shell enclosing the content, wherein the content includes a lipophilic component that is liquid at room temperature and a fat and oil having a slip melting point of 24°C to 55°C,

a storage elastic modulus (G') and a loss elastic modulus (G") obtained by dynamic viscoelasticity measurement of the content satisfy relationships of:

at 25°C, G' > G" and $4.0 \times 10^3$ Pa $\leq$ G' $\leq 2.0 \times 10^5$ Pa;

in a temperature range of 25°C to 40°C, G' and G" decrease; and

at 40°C, G' < G" and both G' and G" are $1.0 \times 10^2$ Pa or less.

Fig. 1

**Description**

TECHNICAL FIELD

The present invention relates to a seamless capsule for an oral cavity which dissolves readily in the oral cavity.

BACKGROUND ART

Various encapsulated medicines, foods, personal preference items, and the like are currently widely used because of their ease of intake. Among them, as a capsule enclosing a liquid content, a seamless capsule manufactured using a droppingin-liquid method is widely used because of high productivity and the like. One example of such seamless capsules is a seamless capsule to be used as a personal preference item or for oral care.

JP 2013-71934 A (Patent Document 1) discloses a seamless capsule including a capsule content solution (content) and a capsule shell enclosing the content, and a method for producing the same. The seamless capsule of Patent Document 1 relates to improvement of a capsule shell, and a dissolution time of the capsule shell is controlled. The content used in Patent Document 1 is one usually used for a seamless capsule, and is described as a liquid substance in Patent Document 1, and a content in which a material that imparts functionality to the liquid substance is blended is described. In a case where the content is a liquid substance, the person feels the presence of an undissolved shell in dissolving the shell in the oral cavity.

PRIOR ART DOCUMENT

PATENT DOCUMENT

Patent Document 1: JP 2013-71934 A

SUMMARY OF THE INVENTION

PROBLEMS TO BE SOLVED BY THE INVENTION

The present invention provides a seamless capsule for an oral cavity which is a seamless capsule comprising a content in the form of semi-solid instead of liquid, and which allows reducing a feeling of undissolved residue of the shell in the oral cavity.

SOLUTIONS TO THE PROBLEMS

The present invention provides the following aspects.

[1] A seamless capsule for an oral cavity, comprising: a content; and a shell enclosing the content, wherein

the content includes a lipophilic component that is liquid at room temperature and a fat and oil having a slip melting point of 24°C to 55°C,
a storage elastic modulus (G') and a loss elastic modulus (G") obtained by dynamic viscoelasticity measurement of the content satisfy relationships of:

at 25°C, G' > G" and $4.0 \times 10^3$ Pa $\leq$ G' $\leq$ $2.0 \times 10^5$ Pa;
in a temperature range of 25°C to 40°C, G' and G" decrease; and
at 40°C, G' < G" and both G' and G" are $1.0 \times 10^2$ Pa or less.

[2] The seamless capsule for an oral cavity according to [1], wherein in the dynamic viscoelasticity measurement of the content, the storage elastic modulus G' satisfies the following relational expression:

$$\{\log(G'(35°C)) - \log(G'(40°C))\} \geq 0.9.$$

[3] The seamless capsule for an oral cavity according to [1] or [2], wherein in the dynamic viscoelasticity measurement of the content, G' decreases at least in the temperature range of 25°C to 40°C.

[4] The seamless capsule for an oral cavity according to any one of [1] to [3], wherein the content further includes a fat and oil having a slip melting point of 60°C or more.

[5] The seamless capsule for an oral cavity according to any one of [1] to [4], wherein the shell has a shell dissolution time of 90 seconds or less at a temperature of 50°C.

[6] The seamless capsule for an oral cavity according to any one of [1] to [5], wherein the content further includes a functional component.

[7] The seamless capsule for an oral cavity according to [6], wherein the functional component is at least one component selected from the group consisting of vitamin A, vitamin D, vitamin E, vitamin B1, vitamin B2, vitamin B6, vitamin B12, vitamin C, folic acid, niacin, pantothenic acid, biotin, caffeine, iron, copper, zinc, manganese, selenium, chromium, molybdenum, polyglutamic acid, vine tea polyphenol, blueberry extract, cassis extract, bilberry extract, Salacia extract, carrot powder, Acanthopanacis Cortex, Glycyrrhiza, Peony Root, Cinnamon Bark, Fennel, Amomum Seed, Siraitia grosvenorii, Bifidobacteria, lactic acid bacteria, and yeast.

EFFECTS OF THE INVENTION

The present invention is a seamless capsule for an oral cavity in which not a liquid content but a semi-solid or viscous sticky content is covered with a shell, and the present invention can provide a seamless capsule for an oral cavity which encloses a content different from that of conventional seamless capsules and can reduce a feeling of undissolved residue of the shell in the oral cavity.

BRIEF DESCRIPTION OF THE DRAWINGS

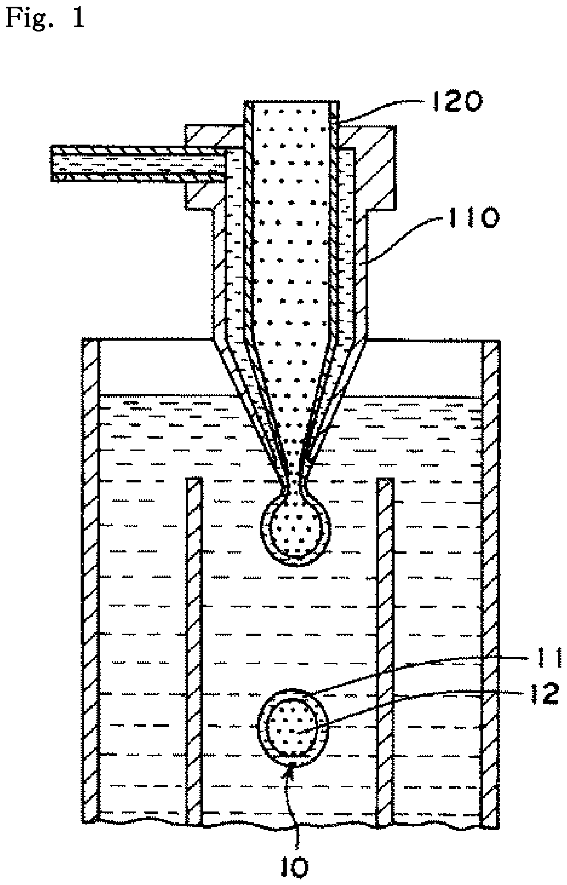

[Fig. 1] Fig. 1 is a schematic vertical sectional view illustrating one embodiment of a nozzle portion of an apparatus for manufacturing a seamless capsule for an oral cavity of the present invention.

DETAILED DESCRIPTION

The present invention provides a seamless capsule for an oral cavity (hereinafter sometimes simply referred to as "seamless capsule") including a content and a shell formed on the content. Referring to Fig. 1, Fig. 1 is a sectional view of a nozzle portion used for forming a seamless capsule 10 for an oral cavity, and a content 12 of the seamless capsule is covered with a shell 11. In the present invention, the seamless capsule is characterized in that the content of the seamless capsule includes a lipophilic component that is liquid at room temperature and a fat and oil having a slip melting point of 24°C to 55°C, and the storage elastic modulus (G') and the loss elastic modulus (G") obtained by dynamic viscoelasticity measurement of the content satisfy relationships of:

at 25°C, $G' > G''$ and $4.0 \times 10^3$ Pa $\leq G' \leq 2.0 \times 10^5$ Pa;
in a temperature range of 25°C to 40°C, G' and G" decrease; and
at 40°C, $G' < G''$ and both G' and G" are $1.0 \times 10^2$ Pa or less.

In the seamless capsule for an oral cavity of the present invention, the shell is illustrated as being of one layer in Fig. 1, but may be of a plurality of layers, namely, two or more layers, and one or a plurality of intermediate layers may, as necessary, be provided between the content and the shell to assist the functions of both layers or, on the contrary, to suppress interaction between both of the layers, but an aspect in which the shell is of one layer and no intermediate layer is present is preferable. That is, in the following description, an aspect of a seamless capsule including two layers, namely, a content and a shell will be mainly described.

<Content>

The "lipophilic component that is liquid at room temperature" used as the content of the seamless capsule for an oral cavity of the present invention is not particularly limited as long as being a lipophilic component that is liquid at room temperature, and examples thereof include a flavor, a grain oil, a fruit oil, and a lipophilic solvent. The lipophilic component preferably includes a flavor, and more preferably includes a flavor and a lipophilic solvent. The lipophilic component may be prepared as an oil/water/oil type emulsion using known materials. The lipophilic component may be used singly or in combination of two or more kinds thereof.

In the present invention, when the seamless capsule for an oral cavity contains a flavor, the feeling of undissolved residue of the shell in the oral cavity can be reduced, and insolubilization of the shell that occurs particularly when a flavor containing a large amount of aldehydes is used can be avoided. The insolubilization of the shell also occurs when the capsule is stored at a high temperature, but the insolubilization reaction can also be suppressed with the content of the present embodiment.

The flavor is not particularly limited as long as it is a lipophilic component, and either a natural flavor or a synthetic flavor may be used. The flavor may be used singly or in combination of two or more kinds thereof. The natural flavor is not particularly limited, and examples thereof include oils such as rose, jasmine, citrus aurantium neroli, chamomile, ylang ylang, geranium, eucalyptus, tea tree, petitgrain, unshiu mikan, orange, lemon, lime, bergamot, pepper, juniper, vanilla, sandalwood, pine, cypress, cinnamon, frankincense, myrrh, vetiver, spike nard, orris root, lavender, lemongrass, basil, rosemary, mints (for example, spearmint, menthol, peppermint, and the like), and berries (for example, blueberry, cranberry, lingonberry, huckleberry, raspberry, blackberry, loganberry, salmonberry, boysenberry, strawberry, bilberry, elderberry, Gooseberry and the like). The synthetic flavor is not particularly limited as long as it is conventionally used for the purpose of imparting aromatic odor and flavor. Examples thereof include esters, alcohols, aldehydes, ketones, phenols, ethers, lactones, hydrocarbons, nitrogen-containing compounds, sulfur-containing compounds, acids, and mixtures thereof.

The esters to be used as the flavor of the lipophilic component are not particularly limited, and examples thereof include propyl formate, butyl formate, amyl formate, octyl formate, linalyl formate, citronellyl formate, geranyl formate, neryl formate, terpinyl formate, ethyl acetate, isopropyl acetate, isoamyl acetate, hexyl acetate, cis-3-hexenyl acetate, trans-2-hexenyl acetate, octyl acetate, nonyl acetate, decyl acetate, dodecyl acetate, dimethyl undecadienyl acetate, styralyl acetate, ocimenyl acetate, myrcenyl acetate, dihydromyrcenyl acetate, linalyl acetate, citronellyl acetate, geranyl acetate, neryl acetate, tetrahydromugol acetate, lavandulyl acetate, nerolidyl acetate, dihydrocuminyl acetate, terpinyl acetate, citryl acetate, nopyl acetate, dihydroterpinyl acetate, 2,4-dimethyl-3-cyclohexenylmethyl acetate, myraldyl acetate, vetiveryl acetate, decenyl propionate, linalyl propionate, geranyl propionate, neryl propionate, terpinyl propionate, tricyclodecenyl propionate, styralyl propionate, anisyl propionate, octyl butyrate, neryl butyrate, cinnamyl butyrate, isopropyl isobutyrate, octyl isobutyrate, linalyl isobutyrate, neryl isobutyrate, linalyl isovalerate, terpinyl isovalerate, phenylethyl isovalerate, 2-methyl pentyl 2-methylvalerate, methyl 3-hydroxyhexanoate, ethyl 3-hydroxyhexanoate, methyl octanoate, octyl octanoate, linalyl octanoate, methyl nonanoate, methyl undecylenate, linalyl benzoate, methyl cinnamate, isoprenyl angelate, methyl geranate, triethyl citrate, ethyl acetoacetate, ethyl 2-hexylacetoacetate, ethyl benzylacetoacetate, allyl 2-ethylbutyrate, ethyl 3-hydroxybutyrate, ethyl nonanoate, ethyl decanoate, ethyl 2,4-decadienoate, propyl 2,4-decadienoate, methyl and linalyl anthranilate, and ethyl N-methylanthranilate.

The alcohols to be used as the flavor of the lipophilic component are not particularly limited, and examples thereof include 3-heptanol, 1-nonanol, n-undecanol, 2-undecanol, n-dodecanol, prenol, 10-undecene-1-ol, dihydrolinalool, tetrahydromugol, myrcenol, dihydromyrcenol, tetrahydromyrcenol, ocimenol, terpineol, hotrienol, 3-thujanol, benzyl alcohol, β-phenylethyl alcohol, α-phenylethyl alcohol, 3-methyl-1-pentanol, 1-heptanol, 2-heptanol, 3-octanol, 1-nonanol, 2-nonanol, 2,6-dimethylheptanol, 1-decanol, trans-2-hexenol, cis-4-hexenol, methyltrimethylcyclopentenyl butenol, citronellol, dihydromyrcenol, rhodinol, geraniol, nerol, linalool, tetrahydrolinalool, dimethyloctanol, hydroxycitronellol, isopulegol, menthol (for example, 1-menthol), terpineol, dihydroterpineol, carveol, dihydrocarbeol, perilla alcohol, 4-thujanol, myrtenol, α-fenchyl alcohol, farnesol, nerolidol, cedrenol, anise alcohol, hydratropic alcohol, 3-phenylpropyl alcohol, cinnamic alcohol, and amyl cinnamic alcohol.

The aldehydes to be used as the flavor of the lipophilic component are not particularly limited, and examples thereof include acetaldehyde, n-hexanal, n-heptanal, n-octanal, n-nonanal, 2-methyloctanal, 3,5,5-trimethylhexanal, decanal, undecanal, 2-methyldecanal, dodecanal, tridecanal, tetradecanal, trans-2-hexenal, trans-4-decenal, cis-4-decenal, trans-2-decenal, 10-undecenal, trans-2-undecenal, trans-2-dodecenal, 3-dodecenal, trans-2-tridecenal, 2,4-hexadienal, 2,4-decadienal, 2,4-dodecadienal, 5,9-dimethyl-4,8-decadienal, citral, dimethyloctanal, α-methylenecitronellal, citronelloxyacetaldehyde, myrtenal, neral, α- or β-sinensal, myrac aldehyde, phenylacetaldehyde, octanal dimethyl acetal, nonanal dimethyl acetal, decanal dimethyl acetal, decanal diethyl acetal, 2-methylundecanal dimethyl acetal, citral dimethyl acetal, citral diethyl acetal, citral propylene glycol acetal, n-valeraldehyde, isovaleraldehyde, 2-methylbutanal, 2-pentenal, trans-2-heptenal, trans-2-nonenal, 2,6-dimethyl-5-heptenal, 2,4-undecadienal, trimethyldecadienal, citronellal, hydroxycitronellal, safranal, vernaldehyde, benzaldehyde, p-isopropylphenylacetaldehyde, p-methylhydratropic aldehyde, phenylpropionaldehyde, 2-methyl-3-(4-methylphenyl)propanal, cyclamenaldehyde, cinnamic aldehyde, salicylaldehyde, anisealdehyde, p-methylphenoxyacetaldehyde, acetaldehyde diethyl acetal, citronellyl methyl acetal, acetaldehyde 2-phenyl-2,4-pentanediol acetal, 2-hexenal diethyl acetal, cis-3-hexenal-diethyl acetal, heptanal diethyl acetal, 2-hexyl-5-methyl-1,3-dioxolane, citronellal cyclomonoglycol acetal, hydroxycitronellal dimethyl acetal, and phenylacetaldehyde dimethyl acetal.

The ketones to be used as the flavor of the lipophilic component are not particularly limited, and examples thereof include 2-pentanone, 3-hexanone, 2-heptanone, 3-heptanone, 4-heptanone, 2-octanone, 3-octanone, 2-nonanone, 2-undecanone, methylheptenone, dimethyloctenone, geranylacetone, farnesylacetone, 2,3,5-trimethyl-4-cyclohexenyl-1-methyl ketone, nerone, nootkatone, dihydronootkatone, acetophenone, 4,7-dihydro-2-isopentyl-2-methyl-1,3-dioxepin, 2-pentanone, 3-hexanone, 2-heptanone, 2,3-hexadione, 3-nonanone, ethyl isoamyl ketone, diacetyl, amyl cyclopentenone, 2-cyclopentyl cyclopentanone, hexylcyclopentanone, heptylcyclopentanone, cis-jasmone, dihydrojasmone, trimethylpentylcyclopentanone, 2-(2-(4-methyl)-3-cyclohexen-1-yl)propylcyclopentanone, damascone, α-dynascone, trimethylcyclohexenylbutenone, α-ionone, β-ionone, methylionone, allylionone, plicatone, cashmeran, 1-carvone, menthone, camphor, p-methylacetophenone, p-methoxyacetophenone, benzylideneacetone, raspberry ketone, methyl naphthyl ketone, benzophenone, furfural acetone, homofuronol, maltol, ethyl maltol, and ethyl acetoacetate ethylene glycol ketal.

The phenols to be used as the flavor of the lipophilic component are not particularly limited, and examples thereof include thymol, carvacrol, β-naphthol isobutyl ether, anethole, β-naphthol methyl ether, β-naphthol ethyl ether, creosol, veratrole, hydroquinone dimethyl ether, 2,6-dimethoxyphenol, 4-ethylguaiacol, eugenol, isoeugenol, ethyl isoeugenol, and tert-butylhydroquinone dimethyl ether.

The ethers to be used as the flavor of the lipophilic component are not particularly limited, and examples thereof include decyl vinyl ether, α-terpinyl methyl ether, isoproxene, 2,2-dimethyl-5-(1-methyl-1-propenyl)-tetrahydrofuran, rosefuran, 1,4-cineol, nerol oxide, 2,2,6-trimethyl-6-vinyl tetrahydropyran, methylhexyl ether, ocimene epoxide, limonene oxide, rhubofix, caryophyllene oxide, linalool oxide, 5-isopropenyl-2-methyl-2-vinyltetrahydrofuran, nerol oxide, and rose oxide.

The lactones to be used as the flavor of the lipophilic component are not particularly limited, and examples thereof include γ-undecalactone, δ-dodecalactone, γ-hexalactone, γ-nonalactone, γ-decalactone, γ-dodecalactone, jasminlactone, methyl-γ-decalactone, 7-decenolactone, jasmolactone, propylidenephthalide, δ-hexalactone, δ-2-decenolactone, ε-dodecalactone, dihydrocoumarin, and coumarin.

The hydrocarbons to be used as the flavor of the lipophilic component are not particularly limited, and examples thereof include ocimene, limonene, α-phellandrene, terpinene, 3-carene, bisabolene, valencene, alloocimene, myrcene, farnesene, α-pinene, β-pinene, camphene, terpinolene, p-cymene, cedrene, β-caryophyllene, and cadinene.

The nitrogen-containing compounds or sulfur-containing compounds to be used as the flavor of the lipophilic component are not particularly limited, and examples thereof include methyl anthranilate, ethyl anthranilate, methyl N-methylanthranilate, methyl N-2'-methylpentylideneanthranilate, ligantral, dodecanenitrile, 2-tridecenenitrile, geranylnitrile, citronellylnitrile, 3,7-dimethyl-2,6-nonadienenitrile, indole, 5-methyl-3-heptanone oxime, limonenethiol, 1-p-menthene-8-thiol, butyl anthranilate, cis-3-hexenyl anthranilate, phenylethyl anthranilate, cinnamyl anthranilate, dimethyl sulfide, and 8-mercaptomenthone.

The "fat and oil having a slip melting point of 24°C to 55°C" to be used for the content of the seamless capsule for an oral cavity of the present invention is merely required to have a slip melting point within a range of 24°C to 55°C among fats and oils, and is used in a sense that a suitable fat and oil is selected from many fats and oils. The "slip melting point" is defined as "temperature at which a sample softens sufficiently and rises in an open capillary tube", and is measured in accordance with ISO 6321:2002. When the slip melting point is in the range of 24°C to 55°C, the feeling of dissolving in the mouth of the content is good.

Fats and oils are glycerin esters of fatty acids, and among them, the fat and oil to be used in the present invention may be any of monoglyceride, diglyceride, and triglyceride as long as the slip melting point is in the range of 24°C to 55°C. The fatty acids that can be used preferably have 10 to 20 carbon atoms. Such fatty acids are, for example, at least one selected from the group consisting of lauric acid (12 carbon atoms), myristic acid (14 carbon atoms), palmitic acid (16 carbon atoms), stearic acid (18 carbon atoms), oleic acid (18 carbon atoms; 1 unsaturated double bond), linoleic acid (18 carbon atoms; 2 unsaturated double bonds), linolenic acid (18 carbon atoms; 3 unsaturated double bonds), arachidic acid (20 carbon atoms), and eicosenoic acid (20 carbon atoms; 1 unsaturated double bond), and an arbitrary fatty acid can be used without being limited to the above.

As described above, the content of the seamless capsule of the present invention includes a lipophilic component that is liquid at room temperature and a fat and oil having a slip melting point of 24°C to 55°C, and the amounts of the respective components contained are 20 parts by weight to 90 parts by weight of the lipophilic component and 10 parts by weight to 80 parts by weight of the fat and oil, preferably 20 parts by weight to 60 parts by weight of the lipophilic component and 40 parts by weight to 80 parts by weight of the fat and oil, more preferably 25 parts by weight or more of the lipophilic component and 75 parts by weight or less of the fat and oil, and further preferably 30 parts by weight or more of the lipophilic component and 70 parts by weight or less of the fat and oil based on 100 parts by weight of the content. If the amount of the lipophilic component is less than 20 parts by weight, a semi-solid content is less likely to be formed, so that the feeling of undissolved residue of the shell readily increases, and the feeling of dissolving in the mouth of the seamless capsule in the oral cavity is prone to be poor. On the other hand, if the lipophilic component is more than 90 parts by weight, the content becomes substantially liquid, so that the undissolved residue of the shell is easily perceived conspicuously. In the seamless capsule for an oral cavity of the present invention, the amount of the lipophilic component contained may be 75 parts by weight or less, or may be 70 parts by weight or less based on 100 parts by weight of the content. The amount of the fat and oil contained may be 20 parts by weight or more, may be 30 parts by weight or more, may be 40 parts by weight or more, or may be 45 parts by weight or more based on 100 parts by weight of the content.

The content of the seamless capsule for an oral cavity of the present invention may further include a fat and oil having a slip melting point of 60°C or more. Blending of such a fat and oil having a high melting point can reduce the blending amount of the entire fats and oils, and further, when the content is exposed in the oral cavity, the presence of a semi-solid content in a state of neither a solid nor a liquid can be felt, but the feeling of undissolved residue of the shell is not felt thereafter. For convenience of explanation, the fat and oil having a slip melting point of 60°C or more is referred to as a second fat and oil, and the fat and oil having a slip melting point of 24 to 55°C is referred to as a first fat and oil in some cases. The slip melting point of the second fat and oil is usually 190°C or less, preferably 150°C or less, more preferably 100°C or less, and still more preferably 80°C or less.

The second fat and oil is a fatty acid glycerin ester similarly to the first fat and oil, but may be at least one selected from the group consisting of a glycerin fatty acid ester other than the fat and oil to be used for the first fat and oil, a polyglycerin fatty acid ester, and beeswax. The polyglycerin fatty acid ester is an esterified product of a fatty acid and polyglycerin formed through condensation of a plurality of glycerins, and the number of glycerin backbones, the number of fatty acids, and the type of fatty acid constituting the polyglycerin are not particularly limited as long as the second fat and oil has a slip melting point of 60°C or more. The fatty acid contained in the fatty acid ester or the polyglycerin fatty acid ester preferably has 10 to 30 carbon atoms. Such a fatty acid is, for example, at least one selected from the group consisting of lauric acid (12 carbon atoms), myristic acid (14 carbon atoms), palmitic acid (16 carbon atoms), palmitoleic acid (16 carbon atoms: 1 unsaturated double bond), stearic acid (18 carbon atoms), oleic acid (18 carbon atoms; 1 unsaturated double bond), linoleic acid (18 carbon atoms; 2 unsaturated double bonds), linolenic acid (18 carbon atoms; 3 unsaturated double bonds), arachidic acid (20 carbon atoms), eicosenoic acid (20 carbon atoms; 1 unsaturated double bond), behenic acid (22 carbon atoms), and lignoceric acid (24 carbon atoms), and an arbitrary fatty acid can be used without being limited to the above.

The second fat and oil can be blended in the content in an amount of 10% by weight or less, and preferably 5% by weight or less. If the amount of the second fat and oil is more than 10% by weight, the content is easily formed as a solid content, so that the feeling of undissolved residue of the content tends to increase, and the feeling of dissolving in the mouth of the seamless capsule in the oral cavity is prone to be poor.

The content of the seamless capsule for an oral cavity of the present invention can, as necessary, further include a functional component that exhibits another function. In this case, the "function" means performance that is expected to exert a physiological activity on a living body when present in the living body. Examples of the functional component is at least one component selected from the group consisting of vitamin A, vitamin D, vitamin E, vitamin B1, vitamin B2, vitamin B6, vitamin B12, vitamin C, folic acid, niacin, pantothenic acid, biotin, caffeine, iron, copper, zinc, manganese, selenium, chromium, molybdenum, polyglutamic acid, vine tea polyphenol, blueberry extract, cassis extract, bilberry extract, Salacia extract, ginseng powder, Acanthopanacis Cortex, Glycyrrhiza, Peony Root, Cinnamon Bark, Fennel, Amomum Seed, Siraitia grosvenorii, Bifidobacteria, lactic acid bacteria, and yeast, and an arbitrary component can be used according to the physiological activity on a living body without being limited to the above.

The functional component can be blended in the content in an amount of 30% by weight or less, preferably 3 to 20% by weight, and more preferably 10 to 15% by weight. If the amount of the functional component is more than 30% by weight, a semi-solid content is less likely to be formed, so that the feeling of dissolving in the mouth of the seamless capsule in the oral cavity is prone to be poor.

In the content of the seamless capsule for an oral cavity of the present invention, an excipient, a stabilizer, a surfactant, an auxiliary agent, a foaming agent, or the like may be further appropriately blended. The amount of such additives is not particularly limited, but it should not be an amount with which the function of the seamless capsule of the present invention is inhibited.

The content of the seamless capsule for an oral cavity of the present invention is formed by mixing the components described above, and the elastic modulus (specifically, storage elastic modulus G' and loss elastic modulus G") of the content measured by dynamic viscoelasticity measurement needs to satisfy the following range:

at 25°C, G' > G" and $4.0 \times 10^3 \, Pa \leq G' \leq 2.0 \times 10^5 \, Pa$;
in a temperature range of 25°C to 40°C, G' and G" decrease; and
at 40°C, G' < G" and both G' and G" are $1.0 \times 10^2 \, Pa$ or less.

Under the condition that the storage elastic modulus G' and the loss elastic modulus G" satisfy the above ranges, when the content is exposed in the oral cavity, it is possible to feel the content in a state of semi-solid which is different from liquid, and it is difficult to feel the undissolved residue of the shell thereafter, and the feeling of dissolving in the mouth is improved. Although not being bound by a specific theory, G' represents a solid-like element of a material, and G" represents a liquid-like element of a material, and it is indicated that a solid feeling is strong at 25°C, whereas a liquid feeling is strengthened at around 40°C after a lapse of time in the mouth, and it is meant that G' and G" both decrease with an increase in temperature and are excellent in a specific range.

Dynamic viscoelasticity measurement is important for the content of the seamless capsule for an oral cavity of the present invention. The measurement is conducted using Rheosol-G5000 manufactured by UBM. As a jig to be used, a cone plate having a diameter of 39.97 mm and 2.022° (an angle between a plate surface described later and an inclined surface of the cone plate) is used with a gap of 0.05 mm (a distance between the plate surface and the apex of the cone plate). The temperature dependence is measured 5 minutes after the thermostatic bath reaches a target temperature by setting the frequency to 1 rad/sec and the strain rate to 0.5% after 1 g of a sample is placed on a plate. Measurement is conducted at temperatures 25°C, 30°C, 35°C, 40°C, and 45°C in order. The frequency dependence is measured at a strain rate of 0.5% and a temperature of 24.5±1.5°C in a frequency range of 0.1 to 10 rad/sec.

In the dynamic viscoelasticity measurement of the content, the storage elastic modulus G' preferably satisfies the following relationship:

$$\{\log(G'(35°C)) - \log(G'(40°C))\} \geq 0.9.$$

The right side of the relational expression is preferably 1.0, more preferably 1.2, still more preferably 1.5, further preferably 1.9, and still further preferably 2.0. The right side of the relational expression may be 4.0. When the above relationship is satisfied, it is possible to achieve a feeling of dissolving in the mouth that makes it difficult to feel an undissolved residue. Although not being bound by a specific theory, this relational expression means that it is expressed that the elasticity decreases near the body temperature (35°C to 40°C), and since the content is present as a semi-solid at 35°C in the temperature near the body temperature and the feeling of undissolved residue of the shell can be reduced, and the content becomes a liquid at 40°C, it is conceived that the larger the value of the above relational expression, the better the dissolution in the mouth. The upper limit of this expression is not necessarily required, but the right side of the relational expression is rarely more than 10. In the above relational expression, $\log(G'(40°C))$ is preferably in the range of -2.0 or more and 2.0 or less.

In the dynamic viscoelasticity measurement of the content of the seamless capsule for an oral cavity of the present invention, G' preferably decreases at least in a temperature range of 25°C to 40°C. As a result of a decrease of the G' of the content depending on the temperature, it is possible to achieve a feeling of dissolving in the mouth that makes it difficult to feel an undissolved residue of the shell.

The seamless capsule for an oral cavity of the present invention is merely required that after the seamless capsule disintegrates in the oral cavity, the feeling of undissolved residue of the shell can be reduced by the content, and the amount of the content is not particularly limited, but the amount of the content can be, for example, within a range of 500 parts by weight or more and 1600 parts by weight or less based on 100 parts by weight of the shell.

<Shell>

In the seamless capsule for an oral cavity of the present invention, the outside of the content is covered with a shell. The shell contains a water-soluble natural polymer. The water-soluble natural polymer is selected from, for example, among gelatin, casein, zein, pectin or derivatives thereof, alginic acid or salts thereof, agar, gellan gum, carrageenan, furcellaran, chitosan, curdlan, starch, modified starch, pullulan, mannan and mixtures thereof. Of course, the water-soluble natural polymer is not limited thereto. These water-soluble natural polymers are present preferably in a range of 50% by weight to 90% by weight based on the total solid weight of the shell composition of the seamless capsule. When an alginate salt, gellan gum, pectin, or carrageenan is used, an alkali metal salt, an alkaline earth metal salt, an ammonium salt, or the like may be optionally added.

The shell of the seamless capsule for an oral cavity of the present invention may further contain a plasticizer to impart flexibility in a dry state, and examples of the plasticizer include glycerin and sorbitol. The blending amount of the plasticizer is 1 to 50% by weight, preferably 5 to 40% by weight, and more preferably 15 to 30% by weight based on the total weight of the shell after drying. If the blending amount of the plasticizer is less than 1% by weight, the shell cannot withstand vacuum drying, or cannot maintain sufficient flexibility in a dry state, so that cracks occur. If the blending amount of the plasticizer is more than 50% by weight, the shell is softened, so that adhesion or melting occurs at a high temperature.

As necessary, the shell of the seamless capsule for an oral cavity of the present invention may contain, in addition to the above composition, various additives commonly used in this field, such as flavors, sweeteners, coloring agents, and preservatives such as paraben. When such additives are used, the total amount of all the additives contained is, for example, 0.01% by weight to 10% by weight, and preferably 0.1% by weight to 5% by weight, based on the total solid weight in the composition to be the shell of the capsule.

It is desirable that the shell of the seamless capsule for an oral cavity of the present invention has a thickness of 10 to 600 μm, preferably 30 to 400 μm, and more preferably 40 to 250 μm. When the thickness of the shell is less than 10 μm, the shell strength tends to be low, and when the thickness exceeds 600 μm, the amount of the content decreases, so that the disintegrability tends to be poor.

The size of the seamless capsule for an oral cavity of the present invention is not particularly limited, and it is desirable that the seamless capsule has a diameter of 0.3 to 10 mm, and preferably 1 to 8 mm. When the diameter of the capsule is less than 0.3 mm, the thickness of the intermediate layer of the layer structure of the seamless capsule is small, so that the effect of preventing entry of moisture tends to be reduced, and when the diameter exceeds 8 mm, the capsule may be too large to swallow in the form of capsule.

The seamless capsule for an oral cavity of the present invention preferably attains a dissolution time of the shell of 90 seconds or less when the seamless capsule is subjected to a dissolution test in a temperature environment of 50°C. The shell dissolution time is more preferably 85 seconds or less, and most preferably 80 seconds or less. When the shell dissolution time at 50°C exceeds 90 seconds, the shell tends to remain in the oral cavity even after the content is dissolved and disappears.

In measuring the dissolution time of the shell, a constant temperature water bath type dissolution tester (for example, NTR-6400A manufactured by Toyama Sangyo Co., Ltd.) is used. As a method for measuring the dissolution time of the shell, 300 mL of purified water is placed in a vessel, and the vessel is placed in a constant temperature water bath type dissolution tester. Next, the temperature in the vessel is set to 50 ± 0.5°C, and the rotation speed of the paddle is set to 250 rpm. Then, the seamless capsule is added into the vessel, and the time taken until the shell is completely dissolved is measured. At that time, it is visually confirmed that no insolubilized product of the shell is present in the vessel. The dissolution time of the shell is measured five times, and the average of the measurements is calculated. When the dissolution time of the shell exceeds 90 seconds, the shell remaining undissolved in the oral cavity is more likely to be felt conspicuously than the content, so that the feeling of dissolving in the mouth of the seamless capsule is prone to be poor.

The seamless capsule for an oral cavity of the present invention is usually spherical, but it is merely required that the shell has no seam, and the shape of the seamless capsule may be a hemispherical shape, a button shape, or an irregular shape.

<Method for producing seamless capsule for oral cavity>

The method for producing the seamless capsule for an oral cavity of the present invention can be conducted with a production apparatus as illustrated in Fig. 1. The production apparatus includes a multilayer nozzle and a cooling tube through which a cooling liquid described later is made to flow down.

In the production of the seamless capsule for an oral cavity of the present invention, a capsule shell preparation liquid and a capsule content liquid are prepared in advance. Then, each of the capsule shell preparation liquid and the capsule content liquid is discharged from the multilayer nozzle to form a composite jet. The tip portion of the composite jet is discharged into the flow path of the cooling tube, whereby a wet capsule (10) is formed. The structure of the wet capsule (10) is similar to that of the seamless capsule, and is a structure in which a content is enclosed. In the process of forming such a wet capsule (10), the capsule content liquid solidifies to become a content, and the capsule shell preparation liquid coagulates while covering the content.

The capsule shell preparation liquid includes a natural polymer and a solvent. The natural polymer is a component that gels the capsule shell preparation liquid, and is often water soluble. One example of the natural polymer is gelatin. As the natural polymer, any component can be applied without being limited to gelatin as long as it does not affect the feeling of dissolving in the mouth of the seamless capsule.

The solvent is a liquid for dispersing components in the capsule shell preparation liquid. Such a solvent is, for example, at least one selected from the group consisting of water and ethanol. Examples of the water include tap water, ion-exchanged water, distilled water, ultrapure water, and mixtures thereof.

The capsule shell preparation liquid may further include a plasticizer. The plasticizer is a component that imparts flexibility to the coagulated product of the capsule shell preparation liquid. The plasticizer can maintain sufficient flexibility of the shell and can make the shell hardly crack particularly in a dry capsule, which is a dried product of a wet capsule. Examples of the plasticizer include polyhydric alcohols such as glycerin and sorbitol. The plasticizer is merely required to be able to impart flexibility to the coagulated product of the capsule shell preparation liquid, and an arbitrary component can be applied without being limited to the above.

The capsule shell preparation liquid may further include an additive such as a colorant, a taste component, a preservative, or a flavor, as necessary.

In the method for producing a seamless capsule for an oral cavity according to one aspect of the present invention, a double nozzle can be used as a multiple nozzle as illustrated in Fig. 1. In one aspect illustrated in Fig. 1, a capsule shell preparation liquid is fed to a first nozzle (120), and a capsule content liquid is fed to a second nozzle (110). Then, these liquids are simultaneously extruded from the tips of the respective annular holes, and the obtained two-phase composite jet is discharged into a cooling liquid flowing down, whereby a wet capsule (10) can be obtained.

This cooling liquid is typically at 20°C or less, and preferably in the range of 1 to 18°C. The temperature of each liquid extruded from the nozzle is not particularly limited, but is typically in the range of 15 to 70°C, and preferably in the range of 20 to 65°C.

As the cooling liquid, for example, medium-chain fatty acid triglycerides (MCT), vegetable fats and oils (coconut oil, sunflower oil, safflower oil, sesame oil, rapeseed oil, grape seed oil, mixtures thereof, and the like), liquid paraffin, and mixtures thereof can be used.

In the above production method, it is also possible to apply appropriate vibration to the composite jet stream using a vibrator to improve cutting of the composite jet, thereby making the particle size uniform, and facilitating encapsulation. It is more preferable that the multiple nozzle is a concentric multiple nozzle.

In the above production method, after the wet capsule (10) is prepared, the wet capsule (10) may be dried to afford a dry capsule. A conventional drying method can be applied to dry the wet capsule (10). However, in the above production method, drying of the wet capsule (10) is not essential, and the wet capsule (10) may be used as the seamless capsule of the present invention.

When the seamless capsule for an oral cavity of the present invention has a three-layer structure, in the composite jet, as a liquid substance for separating the capsule content liquid and the capsule shell preparation liquid, another lipophilic liquid substance or the like is included, for example. The liquid substance for the separation may contain, for example, the active component, and/or flavor, as described the above. In the case of preparing a seamless capsule having a three-layer structure, the seamless capsule can be prepared using the same method as the method described above and illustrated in Fig. 1 except that a concentric triple nozzle in which a nozzle is additionally provided inside the first nozzle (120) (inner nozzle) depicted in Fig. 1 is used. A seamless capsule for an oral cavity having a four-layer structure can also be prepared by a similar method.

The seamless capsule for an oral cavity of the present invention may be subjected to washing with water, heat pasteurization, or heat sterilization, as necessary, according to its use conditions.

[Examples]

The present invention will be described hereafter in more detail by way of examples, to which the present invention is not intended to be limited. In the following examples, all designations of "part(s)" and "%" are on the weight basis, unless otherwise stated.

In Examples and Comparative Examples, the following components were used.

| | |
|---|---|
| · Mint flavor: | Peppermint flavor manufactured by Symrise |
| · Fat and oil A: | H45 (manufactured by Taiyo Yushi Corp., glycerin fatty acid ester (slip melting point: 43.5 to 46.5°C)) |
| · Fat and oil B: | Taiset-50 (manufactured by Taiyo Yushi Corp., mixture of fatty acid ester and polyglycerin fatty acid ester (slip melting point: 72.6°C)) |
| · Beeswax: | Food additive beeswax (G.B) manufactured by Miki Chemical Industry & Co., Ltd., slip melting point: 63.4°C |

Example 1

Content liquid: A capsule content liquid was prepared by heating and mixing 50 parts of mint flavor, 47 parts of a fat and oil A, and 3 parts of beeswax as lipophilic components until the mixture became uniform.

Capsule shell preparation liquid: A capsule shell preparation liquid was prepared by mixing 79.8 parts of pig-origin gelatin (jelly strength: 240 bloom, manufactured by Nitta Gelatin Inc.), 2 parts of glycerin, 18 parts of sorbitol, 0.2 parts of colorant (Food Blue No. 1), and 233.3 parts of ion-exchanged water until the mixture became uniform, and then heating and dissolving the resulting mixture at 60°C. The viscosity of the capsule shell preparation liquid was 59 mPa·s (60°C).

Next, using a seamless capsule producing apparatus (manufactured by Morishita Jintan Co., Ltd.) having a structure as illustrated in Fig. 1, the capsule content liquid and the capsule shell preparation liquid were simultaneously discharged into a cooling oil of 10°C from a second nozzle (110) of a concentric double nozzle and from a first nozzle (120), respectively, whereby a wet capsule having a two-layer structure was formed. The wet capsule was then air dried (20 to 30°C), affording a dry capsule. The dry capsules were dealt with as seamless capsules.

The resulting seamless capsule had a diameter after drying of 8 mm, the weight of the shell based on the total weight of the seamless capsule was 6%, and the weight of the content was 94%.

Example 2

A seamless capsule was prepared by the same procedure as in Example 1 except for changing the amount of the fat and oil A in the content liquid to 48 parts and using 2 parts of the fat and oil B instead of the beeswax.

The resulting seamless capsule had a diameter after drying of 8 mm, the weight of the shell based on the total weight of the seamless capsule was 6%, and the weight of the content was 94%.

Example 3

A seamless capsule was prepared by the same procedure as in Example 1 except that the amounts of the mint flavor and the fat and oil A of the content liquid were changed to 25 parts and 75 parts, respectively, and no beeswax was blended.

The resulting seamless capsule had a diameter after drying of 8 mm, the weight of the shell based on the total weight of the seamless capsule was 6%, and the weight of the content was 94%.

Comparative Example 1

A seamless capsule was prepared by the same procedure as in Example 1 except that a capsule content liquid was prepared using only 100 parts of medium-chain fatty acid triglyceride (MCT).

The resulting seamless capsule had a diameter after drying of 8 mm, the weight of the shell based on the total weight of the seamless capsule was 6%, and the weight of the content was 94%.

Comparative Example 2

A capsule content liquid was prepared by heating and mixing 80 parts of mint flavor and 20 parts of beeswax until the mixture became uniform. Then, preparation of a seamless capsule was attempted by the same procedures as in Example 1 using this capsule content liquid, but a seamless capsule could not be prepared.

Example 4

A seamless capsule was prepared by the same procedure as in Example 1 except that MCT and mint flavor were used as lipophilic components and biotin was used as a functional component, 15 parts of the MCT, 15 parts of the mint flavor, 67 parts of the fat and oil A, and 3 parts of biotin were mixed to prepare a content liquid, and at that time, no beeswax was blended.

The resulting seamless capsule had a diameter after drying of 8 mm, the weight of the shell based on the total weight of the seamless capsule was 6%, and the weight of the content was 94%.

Example 5

A seamless capsule was prepared by the same procedure as in Example 1 except that the amounts of the mint flavor and the fat and oil A of the content liquid were changed to 70 parts and 20 parts, respectively, and the amount of the beeswax was changed to 10 parts.

The resulting seamless capsule had a diameter after drying of 8 mm, the weight of the shell based on the total weight of the seamless capsule was 6%, and the weight of the content was 94%.

Comparative Example 3

A capsule content liquid was prepared by heating and mixing 92 parts of mint flavor and 8 parts of beeswax until the mixture became uniform. Then, using this capsule content liquid, a seamless capsule was prepared by the same procedure as in Example 1.

The resulting seamless capsule had a diameter after drying of 8 mm, the weight of the shell based on the total weight of the seamless capsule was 6%, and the weight of the content was 94%.

Using the seamless capsule content liquid of each of Examples and Comparative Examples, dynamic viscoelasticity measurement was conducted as follows, and the values of the storage elastic modulus G' at 25°C and 40°C, the loss elastic modulus G" at 25°C and 40°C, LogG'(35°C), LogG'(40°C), and {log(G'(35°C)) - log(G'(40°C))} of each seamless capsule content liquid are shown in Tables 1 and 3. In Tables 1 and 3, the composition of the seamless capsule content liquid of each of Examples and Comparative Examples is also shown in the unit of part by weight. Since the content of the seamless capsule was a solidified product of the seamless capsule content liquid, the composition, the storage elastic modulus G', and the loss elastic modulus G" of the content were determined by, for example, dynamic viscoelasticity measurement with the seamless capsule content liquid regarded as the content of the seamless capsule.

<Measurement of dynamic viscoelasticity>

For measurement of dynamic viscoelasticity, Rheosol-G5000 manufactured by UBM was used. As a jig, a cone plate having a diameter of 39.97 mm and 2.022° (an angle between a plate surface described later and an inclined surface of the cone plate) was used with a gap of 0.05 mm (a distance between the plate surface and the apex of the cone plate). The temperature dependence was measured 5 minutes after the thermostatic bath reached a target temperature by setting the frequency to 1 rad/sec and the strain rate to 0.5% after 1 g of a sample was placed on a plate. Measurement was conducted at temperatures 25 ± 1°C, 30 ± 1°C, 35 ± 1°C, 40 ± 1°C, and 45 ± 1°C in order. The frequency dependence was measured at a strain rate of 0.5% and a temperature of 24.5±1.5°C in a frequency range of 0.1 to 10 rad/sec.

In addition, a sensory test on the feeling of dissolving in the mouth of the obtained seamless capsule was conducted as follows. The results are shown in Tables 1 and 3.

<Sensory test of dissolution in mouth>

The sensory test of dissolution in the mouth was conducted by five persons. For the capsules of Examples 1 to 5 and Comparative Examples 1 to 3, a single capsule was put in the mouth, the shell was dissolved, and the feeling of dissolving in the mouth was evaluated after the content was released. The evaluation was conducted according to the following criteria, and the average of five persons was used as the evaluation result.

Criteria for evaluation of the feeling of dissolving in the mouth

○: After the content is released from the capsule, the content has a semi-solid texture, but dissolves quickly and flows easily. In addition, a feeling of undissolved residue of the shell is hardly felt.
△: After the content is released from the capsule, the content has a semi-solid texture, but dissolves slowly and flows easily. In addition, a feeling of undissolved residue of the shell is hardly felt.
×: The content released from the capsule has no semi-solid texture. In addition, a feeling of undissolved residue of the shell is felt.
××: A capsule cannot be formed, and even when only the content is put in the mouth, it remains undissolved.

Using the obtained seamless capsule (in the following dissolution test, it is referred to as "sample"), the dissolution time of the shell was measured by the following method and entered in Tables 1 and 3.

<Dissolution test (measurement of shell dissolution time)>

For the measurement of the dissolution time of the shell, NTR-6400A manufactured by Toyama Sangyo Co., Ltd. was used. 300 mL of purified water was placed in a vessel, the temperature in the vessel was set to 50 ± 0.5°C, and the rotation speed of the paddle was set to 250 rpm. Thereafter, the sample was added into the vessel, and the time taken until the shell of the sample was completely dissolved was measured. At that time, it was visually confirmed that no insolubilized product of the shell was present in the vessel. The dissolution time of the shell was measured five times, and the average of the measurements was calculated.

[Table 1]

| | | Example 1 | Example 2 | Example 3 | Comparative Example 1 | Comparative Example 2 |
|---|---|---|---|---|---|---|
| Content liquid (parts by weight) | MCT Mint flavor | | | | 100 | |
| | | 50 | 50 | 25 | | 80 |
| | Fat and oil A | 47 | 48 | 75 | | |
| | Fat and oil B | | 2 | | | |
| | Beeswax | 3 | | | | 20 |
| | Biotin | | | | | |
| Sensory test (dissolution in mouth) | | ○ | ○ | △ | × | ×× |
| G'(25°C) | | 174255 | 4243 | 77094 | 0.05 | 12045 |
| G'(40°C) | | 0.02 | 19.9 | 27.2 | 0.02 | 276 |
| G"(25°C) | | 8206 | 3063 | 30195 | 10.0 | 10235 |
| G"(40°C) | | 8.96 | 20.0 | 48.9 | 3.28 | 523 |
| log (G'(35°C)) | | 2.66 | 3.28 | 2.35 | -1.40 | 2.93 |

(continued)

|  | Example 1 | Example 2 | Example 3 | Comparative Example 1 | Comparative Example 2 |
|---|---|---|---|---|---|
| log (G'(40°C)) | -1.81 | 1.30 | 1.44 | -1.63 | 2.44 |
| log (G'(35°C)) - log (G'(40°C)) | 4.47 | 1.98 | 0.92 | 0.23 | 0.49 |
| Shell dissolution time (sec) | 84 | 79 | 85 | 83 | N.D. |
| Temperature error: ±1°C | | | | | |

In Table 1, "N.D." indicates unmeasurable.
In the following Table 2, the measured values of G' and G" of Example 1, Example 3, and Comparative Example 2 were entered.

[Table 2]

|  | | Example 1 | | Example 3 | | Comparative Example 2 | |
|---|---|---|---|---|---|---|---|
|  | | G' | G" | G' | G" | G' | G" |
| Temperature (°C) | 25 | 174254.9 | 8205.537 | 77093.78 | 30194.84 | 12044.96 | 10234.74 |
|  | 30 | 2000.619 | 2891.38 | 1083.835 | 1053.046 | 1451.345 | 2992.114 |
|  | 35 | 460.0766 | 762.8183 | 225.5874 | 411.4228 | 849.5519 | 1908.217 |
|  | 40 | 0.015631 | 8.964198 | 27.23839 | 48.89024 | 275.5702 | 522.6782 |
|  | 45 | 0.566401 | 9.596571 | 0.026698 | 7.660855 | 33.08784 | 32.29319 |
| Temperature error: ±1 °C | | | | | | | |

[Table 3]

|  |  | Example 4 | Example 5 | Comparative Example 3 |
|---|---|---|---|---|
| Content liquid (parts by weight) | MCT | 15 | | |
|  | Mint flavor | 15 | 70 | 92 |
|  | Fat and oil A | 67 | 20 | |
|  | Fat and oil B | | | |
|  | Beeswax | | 10 | 8 |
|  | Biotin | 3 | | |
| Sensory test (dissolution in mouth) | | ○ | Δ | × |
| G'(25°C) | | 50874 | 4106 | 2878 |
| G'(40°C) | | 21.8 | 21.48 | 0.01 |
| G"(25°C) | | 14803 | 3817 | 3236 |
| G"(40°C) | | 38.8 | 67.3 | 5.25 |
| log (G'(35°C)) | | 2.56 | 2.38 | -1.43 |
| log (G'(40°C)) | | 1.34 | 1.33 | -1.86 |
| log (G'(35°C)) - log (G'(40°C)) | | 1.22 | 1.05 | 0.43 |
| Shell dissolution time (sec) | | 77 | 87 | 84 |
| Temperature error: ±1°C | | | | |

In the following Table 4, the measured values of G' and G" of Example 2, Example 4, and Example 5 were entered.

[Table 4]

| | | Example 2 | | Example 4 | | Example 5 | |
|---|---|---|---|---|---|---|---|
| | | G' | G" | G' | G" | G' | G" |
| Temperature (°C) | 25 | 4243.21 | 3062.68 | 50873.96 | 14803.43 | 4105.65 | 3817.164 |
| | 30 | 3298.82 | 3428.61 | 4329.17 | 4803.43 | 1143.21135 | 1229.81 |
| | 35 | 1891.103 | 2428.212 | 363.2331 | 864.5579 | 238.5437 | 352.7915 |
| | 40 | 19.93414 | 20.04582 | 21.77814 | 38.75088 | 21.47587 | 67.347523 |
| | 45 | 0.06358 | 10.8852 | 0.2755013 | 10.96851 | 0.01883931 | 10.485927 |
| Temperature error: ±1°C | | | | | | | |

In the following Table 5, the measured values of G' and G" of Comparative Example 1 and Comparative Example 3 were entered.

[Table 5]

| | | Comparative Example 1 | | Comparative Example 3 | |
|---|---|---|---|---|---|
| | | G' | G" | G' | G" |
| Temperature (°C) | 25 | 0.0524258 | 10.00886 | 2878.003 | 3236.061 |
| | 30 | 0.028106 | 10.96851 | 184.8787 | 219.7368 |
| | 35 | 0.0398903 | 10.21571 | 0.037122 | 26.41624 |
| | 40 | 0.02361 | 3.279349 | 0.0136718 | 5.253746 |
| | 45 | 0.00648 | 1.241016 | 0.0098756 | 3.782115 |
| Temperature error: ±1°C | | | | | |

In Examples, seamless capsules in which the storage elastic modulus G' and the loss elastic modulus G" of the content were within the ranges of the present invention and which were excellent in the feeling of dissolving in the mouth in the sensory test were obtained. On the other hand, in Comparative Example 1, since the content is only medium-chain fatty acid triglyceride (MCT), and G' and G" satisfied G' < G" and G' < 4000 Pa at 25°C, the feeling of dissolving in the mouth in the sensory test was poor, and the undissolved residue of the shell was felt. In Comparative Example 2, the content contained mint flavor and beeswax, and as to G' and G", at 25°C, G' > G" and 4,000 < G' < 20,000 Pa, and at 40°C, G' < G" but both G' and G" exceeded 100 and, therefore, did not satisfy the range of the present invention, and further, seamless capsules could not be prepared. In Comparative Example 3, the content contained mint flavor and beeswax, and as to G' and G", at 25°C, G' < G" and G' < 4000 Pa, and therefore, the range of the present invention was not satisfied, and in the sensory test, it was evaluated "The content released from the capsule has no semi-solid texture. Moreover, a feeling of undissolved residue of the shell is felt." In addition, the beeswax used alone in Comparative Examples 2 and 3 had a slip melting point of 63.4°C, which exceeds the reference value 50°C of the present invention.

As described above, the technology described in the present description includes the following aspects.

[1] A seamless capsule for an oral cavity, comprising: a content; and a shell enclosing the content, wherein

the content includes a lipophilic component that is liquid at room temperature and a fat and oil having a slip melting point of 24°C to 55°C,
a storage elastic modulus (G') and a loss elastic modulus (G") obtained by dynamic viscoelasticity measurement of the content satisfy relationships of:

at 25°C, G' > G" and $4.0 \times 10^3$ Pa $\leq$ G' $\leq 2.0 \times 10^5$ Pa;
in a temperature range of 25°C to 40°C, G' and G" decrease; and
at 40°C, G' < G" and both G' and G" are $1.0 \times 10^2$ Pa or less.

[2] The seamless capsule for an oral cavity according to [1], wherein in the dynamic viscoelasticity measurement of the content, the storage elastic modulus G' satisfies the following relational expression:

# EP 4 785 934 A1

$$\{\log(G'(35°C)) - \log(G'(40°C))\} \geq 0.9.$$

[3] The seamless capsule for an oral cavity according to [1] or [2], wherein in the dynamic viscoelasticity measurement of the content, G' decreases at least in the temperature range of 25°C to 40°C.

[4] The seamless capsule for an oral cavity according to any one of [1] to [3], wherein the content further includes a fat and oil having a slip melting point of 60°C or more.

[5] The seamless capsule for an oral cavity according to any one of [1] to [4], wherein the shell has a shell dissolution time of 90 seconds or less at a temperature of 50°C.

[6] The seamless capsule for an oral cavity according to any one of [1] to [5], wherein the content further includes a functional component.

[7] The seamless capsule for an oral cavity according to [6], wherein the functional component is at least one component selected from the group consisting of vitamin A, vitamin D, vitamin E, vitamin B1, vitamin B2, vitamin B6, vitamin B12, vitamin C, folic acid, niacin, pantothenic acid, biotin, caffeine, iron, copper, zinc, manganese, selenium, chromium, molybdenum, polyglutamic acid, vine tea polyphenol, blueberry extract, cassis extract, bilberry extract, Salacia extract, carrot powder, Acanthopanacis Cortex, Glycyrrhiza, Peony Root, Cinnamon Bark, Fennel, Amomum Seed, Siraitia grosvenorii, Bifidobacteria, lactic acid bacteria, and yeast.

[8] The seamless capsule for an oral cavity according to any one of [1] to [7], wherein an amount of the content is in a range of 500 parts by weight or more and 1600 parts by weight or less based on 100 parts by weight of the shell.

[9] The seamless capsule for an oral cavity according to any one of [1] to [8], wherein the amount of the fat and oil having a slip melting point of 24°C to 55°C contained is 10 parts by weight or more based on 100 parts by weight of the content.

[10] The seamless capsule for an oral cavity according to any one of [1] to [9], wherein the amount of the fat and oil having a slip melting point of 24°C to 55°C contained is 80 parts by weight or less based on 100 parts by weight of the content.

[11] The seamless capsule for an oral cavity according to any one of [1] to [10], wherein the amount of the fat and oil having a slip melting point of 24°C to 55°C contained is 40 parts by weight or more based on 100 parts by weight of the content.

[12] The seamless capsule for an oral cavity according to any one of [1] to [11], wherein the amount of the lipophilic component contained is 20 parts by weight or more based on 100 parts by weight of the content.

[13] The seamless capsule for an oral cavity according to any one of [1] to [12], wherein the amount of the lipophilic component contained is 90 parts by weight or less based on 100 parts by weight of the content.

[14] The seamless capsule for an oral cavity according to any one of [1] to [13], wherein the amount of the lipophilic component contained is 60 parts by weight or less based on 100 parts by weight of the content.

REFERENCE SIGNS LIST

10:     Seamless capsule
11:     Capsule shell
12:     Capsule content
110:    Second nozzle
120:    First nozzle

## Claims

1.  A seamless capsule for an oral cavity, comprising: a content; and a shell enclosing the content, wherein

    the content includes a lipophilic component that is liquid at room temperature and a fat and oil having a slip melting point of 24°C to 55°C,
    a storage elastic modulus (G') and a loss elastic modulus (G") obtained by dynamic viscoelasticity measurement of the content satisfy relationships of:

    at 25°C, G' > G" and $4.0 \times 10^3$ Pa $\leq$ G' $\leq 2.0 \times 10^5$ Pa;
    in a temperature range of 25°C to 40°C, G' and G" decrease; and
    at 40°C, G' < G" and both G' and G" are $1.0 \times 10^2$ Pa or less.

2. The seamless capsule for an oral cavity according to claim 1, wherein in the dynamic viscoelasticity measurement of the content, the storage elastic modulus G' satisfies the following relational expression:

$$\{\log(G'(35°C)) - \log(G'(40°C))\} \geq 0.9.$$

3. The seamless capsule for an oral cavity according to claim 1 or 2, wherein in the dynamic viscoelasticity measurement of the content, G' decreases at least in the temperature range of 25°C to 40°C.

4. The seamless capsule for an oral cavity according to any one of claims 1 to 3, wherein the content further includes a fat and oil having a slip melting point of 60°C or more.

5. The seamless capsule for an oral cavity according to any one of claims 1 to 4, wherein the shell has a shell dissolution time of 90 seconds or less at a temperature of 50°C.

6. The seamless capsule for an oral cavity according to any one of claims 1 to 5, wherein the content further includes a functional component.

7. The seamless capsule for an oral cavity according to claim 6, wherein the functional component is at least one component selected from the group consisting of vitamin A, vitamin D, vitamin E, vitamin B1, vitamin B2, vitamin B6, vitamin B12, vitamin C, folic acid, niacin, pantothenic acid, biotin, caffeine, iron, copper, zinc, manganese, selenium, chromium, molybdenum, polyglutamic acid, vine tea polyphenol, blueberry extract, cassis extract, bilberry extract, Salacia extract, carrot powder, Acanthopanacis Cortex, Glycyrrhiza, Peony Root, Cinnamon Bark, Fennel, Amomum Seed, Siraitia grosvenorii, Bifidobacteria, lactic acid bacteria, and yeast.

Fig. 1

## INTERNATIONAL SEARCH REPORT

| International application No. |
|---|
| **PCT/JP2024/034124** |

### A. CLASSIFICATION OF SUBJECT MATTER

*A61K 9/48*(2006.01)i; *A23L 5/00*(2016.01)i; *A23L 33/10*(2016.01)i; *A61K 47/44*(2017.01)i
FI: A61K9/48; A61K47/44; A23L5/00 C; A23L33/10

According to International Patent Classification (IPC) or to both national classification and IPC

### B. FIELDS SEARCHED

Minimum documentation searched (classification system followed by classification symbols)

A61K9/48; A23L5/00; A23L33/10; A61K47/44

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| X | WO 2019/111711 A1 (MORISHITA JINTAN CO.) 13 June 2019 (2019-06-13) claims 1-6, examples, paragraph [0017] | 1-3, 6-7 |
| Y | | 1-7 |
| X | WO 2019/111398 A1 (MORISHITA JINTAN CO.) 13 June 2019 (2019-06-13) claims 1-10, examples, paragraph [0017] | 1-3, 6-7 |
| Y | | 1-7 |
| X | WO 2020/251039 A1 (MORISHITA JINTAN CO.) 17 December 2020 (2020-12-17) claims, examples 1-4, paragraphs [0013], [0015], [0018] | 1-3, 6-7 |
| Y | | 1-7 |
| Y | JP 2020-83806 A (FANCL CORP.) 04 June 2020 (2020-06-04) paragraphs [0011], [0030], claims | 1-7 |
| Y | JP 2020-105078 A (FANCL CORP.) 09 July 2020 (2020-07-09) paragraph [0022], claims | 1-7 |

☑ Further documents are listed in the continuation of Box C.  ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **11 December 2024** | **24 December 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**EP 4 785 934 A1**

**INTERNATIONAL SEARCH REPORT**

| International application No. |
|---|
| **PCT/JP2024/034124** |

**C.    DOCUMENTS CONSIDERED TO BE RELEVANT**

| Category* | Citation of document, with indication, where appropriate, of the relevant passages | Relevant to claim No. |
|---|---|---|
| Y | JP 2022-159397 A (MORISHITA JINTAN CO.) 17 October 2022 (2022-10-17)<br>paragraphs [0012]-[0023] | 1-7 |

**INTERNATIONAL SEARCH REPORT**
**Information on patent family members**

International application No.

**PCT/JP2024/034124**

| Patent document cited in search report | | | Publication date (day/month/year) | Patent family member(s) | | | Publication date (day/month/year) |
|---|---|---|---|---|---|---|---|
| WO | 2019/111711 | A1 | 13 June 2019 | US 2020/0236984 A1 claims 1-6, examples, paragraph [0041] EP 3721718 A1 CN 111132560 A KR 10-2020-0096202 A | | | |
| WO | 2019/111398 | A1 | 13 June 2019 | US 2020/0236984 A1 claims 1-10, examples, paragraph [0041] EP 3721718 A1 CN 111132560 A KR 10-2020-0096202 A | | | |
| WO | 2020/251039 | A1 | 17 December 2020 | US 2022/0226252 A1 claims, examples 1-4, paragraphs [0031], [0034], [0036] EP 3984530 A1 CN 113924083 A KR 10-2022-0020800 A | | | |
| JP | 2020-83806 | A | 04 June 2020 | (Family: none) | | | |
| JP | 2020-105078 | A | 09 July 2020 | CN 111358000 A | | | |
| JP | 2022-159397 | A | 17 October 2022 | US 2024/0180219 A1 paragraphs [0043]-[0057] EP 4316472 A1 CN 117120042 A KR 10-2023-0163395 A | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013071934 A